# EUROPÄISCHE PATENTANMELDUNG

(11) **EP 0 537 493 A2**
(43) Veröffentlichungstag der Anmeldung: **21.04.1993**
(21) Anmeldenummer: 92115900.0
(22) Anmeldetag: 17.09.1992
(51) Int. Cl.: A61B 17/04

(54) **Medizinisches Instrument zum Manipulieren eines Fadens**

(30) Priorität: 14.10.1991 DE 4133966
(71) Anmelder: Richard Wolf GmbH, D-75438 Knittlingen (DE)
(72) Erfinder: Boebel, Manfred, W-7136 Oetisheim (DE); Messroghli, Hossein, Dr., W-6080 Gross Gerau (DE)
(74) Vertreter: Vollmann, Heiko, Dipl.-Ing.

(57) **Zusammenfassung**

Das medizinische Instrument ist zum Manipulieren eines innerhalb eines Kωrpers, insbesondere einer Kωrperhωhle befindlichen Fadens vorgesehen. Es weist einen Schaft (2) mit einer Handhabe (1) am proximalen Schaftende und zwei Fadenaufnahmen (5,6) am distalen Schaftende (4) auf. Jede Fadenaufnahme (5,6) wird durch eine offene, den Faden zumindest teilweise umgebende F}hrung gebildet, die aus Draht bestehen kann. Die Aufnahmeωffnungen der beiden Fadenaufnahmen (5,6) sind entgegengerichtet angeordnet, so daß mit dem Instrument der Faden sowohl gezogen als auch geschoben werden kann.

## Beschreibung

Die Erfindung betrifft ein medizinisches Instrument zum Manipulieren eines innerhalb eines Körpers, insbesondere einer Körperhöhle, befindlichen Fadens, mit einem Schaft, mit einer Handhabe am proximalen Schaftende und mit einer Fadenaufnahme am distalen Schaftende.

Derartige medizinische Instrumente werden beispielsweise bei der Ligatur eines Blutgefäßes, Organs oder dergleichen eingesetzt. Bei solchen medizinischen - in der Regel endoskopischen - Eingriffen wird zunächst über einen ersten Einstichkanal ein Faden bzw. eine Schlinge in den Körper bzw. in die Körperhöhle eingebracht. Dieser Faden wird dann mittels des hier in Rede stehenden medizinischen Instrumentes (Fadenmanipulator) an der gewünschten Stelle innerhalb der Körperhöhle plaziert. Der Fadenmanipulator wird in der Regel über einen weiteren Einstichkanal in die Körperhöhle eingeführt, beispielsweise über den Instrumentenkanal eines Endoskops oder eine anderweitige Einführhülse.

Als Fadenmanipulator werden nach dem Stand der Technik in der Regel Faßzangen verwendet. Diese weisen zwei Handhaben am proximalen Ende auf, die über einen Schaft mit darin angeordneter Betätigungsvorrichtung mit dem am distalen Ende des Schaftes angeordneten Maulteil in Verbindung stehen. Die Handhabung eines Fadens oder einer Schlinge innerhalb der Körperhöhle mit derartigen Faßzangen gestaltet sich unter Umständen problematisch. Denn das an sich relativ eigensteife Fadenmaterial wird beim Einführen in die Körperhöhle mit Flüssigkeit benetzt und wird durch die Feuchtigkeitsaufnahme dann sehr labil. Es wird zudem empfindlich gegen plastische Verformungen und Beschädigungen, wodurch die garantierten Festigkeitswerte des Fadenmaterials bis zur Unbrauchbarkeit beeinträchtigt werden können. Da die Maulkraft bei derartigen Faßzangen in der Regel nicht begrenzt ist, kann es in der Praxis leicht zu Beschädigungen des Fadens kommen. Im übrigen erfordert die Handhabung der Faßzange einiges Geschick, da zwei Handhaben zu bedienen sind. Dies erfordert insbesondere beim Drehen des Fadens ein umständliches Umgreifen an den Handhaben der Faßzange.

Ausgehend von diesem Stand der Technik liegt der Erfindung die Aufgabe zugrunde, ein gattungsgemäßes medizinisches Instrument zum Manipulieren eines innerhalb des Körpers befindlichen Fadens so auszubilden, daß eine einfache und sichere Handhabung gewährleistet wird, ohne daß die Gefahr einer Beschädigung des Fadens besteht.

Diese Aufgabe wird gemäß der Erfindung dadurch gelöst, daß am distalen Schaftende des Instrumentes zwei Fadenaufnahmen vorgesehen sind, die jeweils durch eine offene und den Faden zumindest teilweise umgebende Führung gebildet und deren Aufnahmeöffnungen entgegengerichtet angeordnet sind.

Mit den entgegengerichteten Aufnahmeöffnungen können sowohl Schub- als auch Zugkräft auf den Faden ausgeübt werden. In Verbindung mit der teilweisen, vorzugsweise den Faden um mindestens 180° umgebenden Führung ist eine einfache und zugleich sichere Manipulation des Fadens möglich. Der erfindungsgemäße Fadenmanipulator ist mit nur einer Handhabe zu bedienen. Die Bedienung ist besonders einfach, da mit dieser Handhabe das gesamte Instrument um seine Längsachse gedreht und/oder in Achsrichtung verschoben werden kann. Eine Verletzung des zu manipulierenden Fadens durch das Instrument ist bei entsprechender Ausgestaltung praktisch nicht möglich, da der Faden innerhalb der Aufnahmen nur geführt wird, jedoch keine seitlichen Druckkräfte auf den Faden ausgeübt werden, wie dies beispielsweise bei einer Zange der Fall ist. Durch den konstruktiv denkbar einfachen Aufbau des erfindungsgemäßen Instrumentes ergeben sich zudem erhebliche Einsparungen in den Fertigungskosten verglichen mit Instrumenten nach dem Stand der Technik. Die erfindungsgemäße Aufnahme kann in einfachster Form aus einem entsprechend geformten, den Schaft distalwärts verlängernden Draht gebildet sein.

Die Unteransprüche kennzeichnen vorteilhafte Ausgestaltungen des erfindungsgemäßen Instrumentes. So ist es von Vorteil, die Fadenaufnahmen als hakenförmige Gebilde auszugestalten, da hierdurch einerseits eine sichere Führung durch ausreichendes Umgreifen des Fadens und andererseits ein problemloses Loslassen des Fadens ermöglicht wird.

Bei der Bildung der Fadenaufnahmen aus Draht kann der Schaft kontinuierlich in den Drahtquerschnitt übergehen, so daß ein am Schaft langlaufender Faden praktisch selbsttätig beim axialen Verschieben des Schaftes in eine der Aufnahmen geführt wird.

Die Aufnahmeöffnungen sind vorzugsweise parallel zur Schaftachse angeordnet, so daß bei Bewegung des Schaftes in Achsrichtung ein vor dem Instrument oder am Instrument liegender Faden in die Aufnahme geführt wird.

Eine bevorzugte Ausbildung des Instrumentes besteht in der Anordnung eines S-förmigen Drahtes am Schaftende. Je nach Anwendungsfall können auch andere Ausbildungen zweckmäßig sein, beispielsweise ein etwa C-förmig gebogener Draht am Schaftende. Insbesondere für solche Anwendungen, bei denen der Faden unter mehrfachem Richtungswechsel innerhalb der Körperhöhle plaziert werden muß, empfiehlt es sich, die Aufnahmen als eine Art Öse auszubilden, so daß der darin befindliche Faden von beiden Aufnahmen vollständig umfangseitig umgriffen wird. Zum Einlassen bzw. Auslassen des Fadens muß dann das Instrument leicht gedreht und axial verschoben werden, um den Faden durch die in einer anderen Ebene befindliche Öffnung zu führen. Hierbei hat der die Aufnahmen bildende Draht am distalen Schaftende einen etwa schraubenlinienförmigen Verlauf.

Vorteilhaft bei allen Ausführungen ist es, die Aufnahmen so anzuordnen, daß sie innerhalb der in Achsrichtung des Schaftes gesehenen Außenkontur desselben liegen. Hierdurch wird ein problemloses Durchführen der Aufnahmen durch ein Einführinstrument gewährleistet. Es besteht beim Einführen nicht die Gefahr der Beschädigung der Aufnahmen, da bereits nach kurzer Einführstrecke, wenn der Schaft zur Anlage an die Einfüllhülse kommt, die Aufnahmen weitestgehend unbelastet sind.

Die Erfindung ist nachfolgend anhand eines in der Zeichnung dargestellten Ausführungsbeispiels näher erläutert. Es zeigen:
- Figur 1: eine Seitenansicht eines erfindungsgemäßen Fadenmanipulators,
- Figur 2: das distale Ende des in Figur 1 dargestellten Fadenmanipulators in vergrößerter Darstellung,
- Figur 3: das distale Ende einer weiteren Ausführung eines Fadenmanipulators in Darstellung nach Figur 2,
- Figur 4: das distale Ende einer dritten Ausführung eines Fadenmanipulators in Darstellung nach Figur 2 und
- Figur 5: eine Ansicht in Richtung des Pfeiles V in Figur 4.

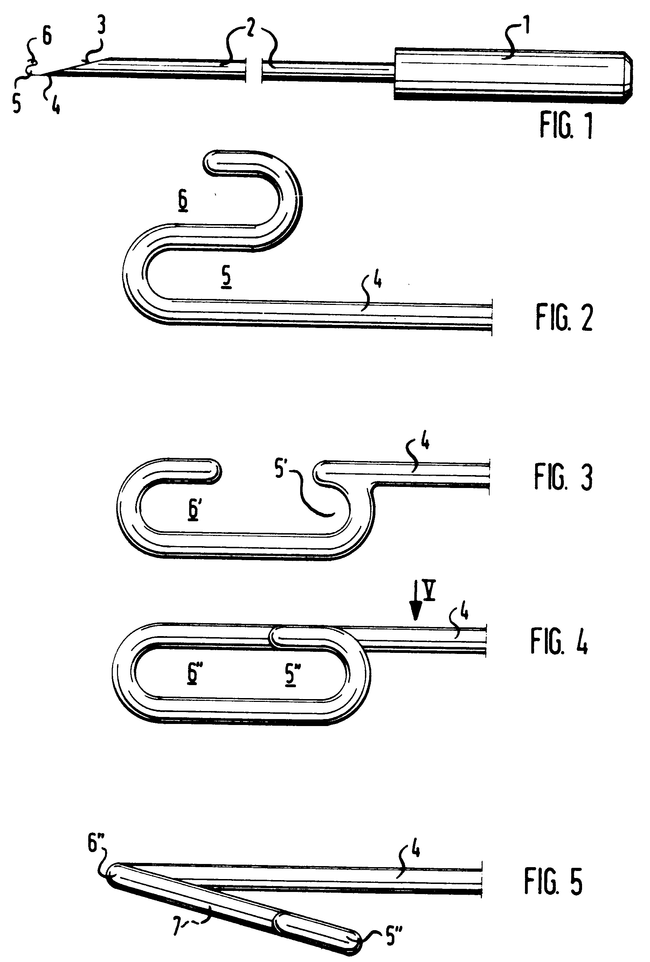

Der in Figur 1 dargestellte Fadenmanipulator weist eine langgestreckte, etwa zylindrische Handhabe 1 auf, die das proximale Ende eines Schaftes 2 bildet und fest mit diesem verbunden ist.

Der Schaft 2 ist in seinem distalen Endbereich 3 zu seinem distalen Ende 4 hin stetig verjüngt ausgebildet. Die Verjüngung erfolgt dergestalt, daß das distale Schaftende 4 exzentrisch innerhalb der Außenkontur des übrigen Schaftes liegt.

An diesem distalen Schaftende 4 sind zwei Fadenaufnahmen 5 und 6 angeordnet, die durch ein S-förmiges, am Schaftende 4 angeformtes Drahtstück gebildet sind. Die Aufnahmen 5,6 bestehen jeweils aus einer Führung in Form eines den Faden teilweise umgreifenden Gebildes. Sie weisen entgegengerichtete Öffnungen auf und sind nebeneinander angeordnet, wie in Figur 2 dargestellt ist. So ist die Aufnahme 5 in Richtung zum proximalen Schaftende und die Aufnahme 6 in Gegenrichtung offen ausgebildet. Es kann beispielsweise mit der in Figur 2 dargestellten Aufnahme 5 ein darin befindlicher Faden in Richtung zum proximalen Instrumentenende gezogen und mit der Aufnahme 6 in Gegenrichtung geschoben werden. Da der Umgreifungswinkel der Aufnahmen etwa 180° betragt und die seitlichen, parallel zum Schaft 2 liegenden Aufnahmeenden verlängert ausgebildet sind, ergibt sich auch eine sichere Seitenführung des Fadens, ohne daß Probleme beim Lösen des Fadens aus dem Manipulator bestehen.

Figur 3 zeigt eine weitere Ausführung, bei der die Aufnahmen 5' und 6' nicht wie beim vorhergehenden Beispiel nebeneinander, sondern gegenüberliegend angeordnet sind. Das am distalen Schaftende 4 angeordnete Drahtstück ist hier etwa C-förmig ausgebildet, so daß ein Faden nach dem Durchtritt aus der jeweiligen Aufnahmeöffnung der Aufnahmen 5' und 6' seitlich entfernt werden kann.

Eine dritte Ausführungsform ist anhand der Figuren 4 und 5 dargestellt. Die dort dargestellten Aufnahmen 5'' und 6'' sind wie bei der Ausführung nach Figur 3 gegenüberliegend mit zueinanderweisenden Öffnungen ausgebildet jedoch münden die jeweiligen Öffnungen der Aufnahmen 5'', 6'' unmittelbar in die Öffnung der gegenüberliegenden Aufnahme 6'' bzw. 5'', so daß der Faden beim Richtungswechsel nicht aus dem Instrument heraus, sondern in die andere Aufnahme gelangt. Die Ein- und Auslaßöffnung 7 ist dadurch gebildet, daß der die Aufnahmen 5'' und 6'' bildende Draht etwa schraubenlinienförmig angeordnet ist, so daß sich eine Öffnung 7 zwischen den durch den Draht aufgespannten Ebenen ergibt.

Alle vorbeschriebenen Ausführungen sind im Bereich ihrer Aufnahmen so ausgebildet, daß diese innerhalb der Außenkontur des Schaftes 2 (in Achsrichtung des Schaftes 2 gesehen) liegen. Hierdurch wird ein problemloses Durchführen durch ein Einführinstrument, beispielsweise ein Endoskop gewährleistet.

Die vorstehend beschriebenen Aufnahmen sind aus Draht gefertigt, es versteht sich, daß diese auch aus Blechen geformt oder anderweitig, beispielsweise durch Gießen gebildet sein können.

## Patentansprüche

1. Medizinisches Instrument zum Manipulieren eines innerhalb eines Körpers, insbesondere einer Körperhohle, befindlichen Fadens, mit einem Schaft (2), mit einer Handhabe (1) am proximalen Schaftende und mit einer Fadenaufnahme (5,6) am distalen Schaftende (4), dadurch gekennzeichnet, daß eine Fadenaufnahme (5,6) durch eine offene und den Faden zumindest teilweise umgebende Führung gebildet ist und daß zwei solcher Fadenaufnahmen (5 und 6) am Schaftende (4) vorgesehen sind, deren Aufnahmeöffnungen entgegengerichtet angeordnet sind.

2. Medizinisches Instrument nach Anspruch 1, dadurch gekennzeichnet, daß die Fadenaufnahmen (5,6) hakenförmige Gebilde sind.

3. Medizinisches Instrument nach einem der vorhergehenden Ansprüche, dadurch gekennzeichnet, daß die Fadenaufnahmen (5,6) aus Draht gebildet sind.

4. Medizinisches Instrument nach einem der vorhergehenden Ansprüche, dadurch gekennzeichnet, daß die Aufnahmeöffnungen in Richtung parallel zur Schaftachse liegen.

5. Medizinisches Instrument nach einem der vorhergehenden Ansprüche, dadurch gekennzeichnet, daß die Aufnahmen (5,6) aus einem S-förmigen Gebilde bestehen, das mit einem Ende stetig in das distale Schaftende (4) übergeht.

6. Medizinisches Instrument nach einem der vorhergehenden Ansprüche, dadurch gekennzeichnet, daß die Aufnahmen (5,6) in Achsrichtung des Schaftes (2) gesehen innerhalb der Außenkontur desselben liegen.

7. Medizinisches Instrument nach einem der vorhergehenden Ansprüche, dadurch gekennzeichnet, daß die Aufnahmeöffnungen (5',6';5'',6'') gegenüberliegend angeordnet sind.

8. Medizinisches Instrument nach einem der vorhergehenden Ansprüche, dadurch gekennzeichnet, daß die beiden Aufnahmen (5'',6'') zum vollständigen Umgreifen eines Fadens ausgebildet sind, wobei sich die Führungen zur Bildung einer Ein- und Auslaßöffnung (7) für den Faden über mehrere Ebenen quer zur Fadenachse erstrecken.
